# EUROPEAN PATENT SPECIFICATION

(11) **EP 3 435 917 B1**
(45) Date of publication and mention of the grant of the patent: **24.04.2024**
(21) Application number: 17716711.1
(22) Date of filing: 28.03.2017
(51) Int. Cl.: A61F 2/07

(54) **ENDOLUMINAL PROSTHETIC DEVICES HAVING FLUID-ABSORBABLE COMPOSITIONS FOR REPAIR OF A VASCULAR TISSUE DEFECT**
ENDOLUMINALE PROTHESEN MIT FLÜSSIGKEITSABSORBIERBAREN ZUSAMMENSETZUNGEN ZUR REPARATUR EINES VASKULÄREN GEWEBEDEFEKTS
DISPOSITIFS PROTHÉTIQUES ENDOLUMINAUX AYANT DES COMPOSITIONS RÉSORBABLES PAR FLUIDE POUR LA RÉPARATION D'UN DÉFAUT DE TISSU VASCULAIRE

(30) Priority: 31.03.2016 US 201662316395 P
(43) Date of publication of application: 06.02.2019
(73) Proprietor: Medtronic Vascular Inc., Santa Rosa, CA 95403 (US)
(72) Inventor: PERKINS, Keith, Santa Rosa, CA 95403 (US); PETRUSKA, Matthew, Santa Rosa, CA 95403 (US); ROBAINA, Samuel, Santa Rosa, CA 95403 (US); GALLIGAN, Darren, Santa Rosa, CA 95403 (US); RADHAKRISHNAN, Rajesh, Santa Rosa, CA 95403 (US)
(74) Representative: Zimmermann & Partner Patentanwälte mbB
(86) International application number: PCT/US2017/024496
(87) International publication number: WO 2017/172735

(56) References cited:
- WO-A1-99/39662
- WO-A2-03/003945
- US-A1- 2006 149 364

## Description

### CROSS-REFERENCE TO RELATED APPLICATIONS

The present application claims the benefit of prior U.S. Appl. No. 62/316,395, filed March 31, 2016.

### FIELD OF THE INVENTION

The present technology relates generally to endoluminal prosthetic devices for repair of vascular tissue defects. In particular, several embodiments are directed to systems and devices to treat a blood vessel defect, such as an aneurysm, a dissection, a penetrating ulcer and/or a traumatic transection, in an aorta of a patient.

### BACKGROUND OF THE INVENTION

Tissue defects within blood vessels, such as aneurysms (e.g., aortic aneurysms) or dissections, for example, can lead to pain (e.g., abdominal and back pain), stroke and/or eventual ruptures in the vessel. Aneurysms occur when there is a weakening in the wall of the blood vessel leading to a widening, opening or formation of a cavity within the vessel wall. The opening of such a cavity can be further exasperated by the continual interrogation from blood pooling in the cavity pressurizing the already weakened vessel wall. Such a damaged vessel, which can be age-related, drug or tobacco-induced, resulting from atherosclerosis or in some instances, or caused by infection, can result in a vessel rupture leading to life-threatening internal bleeding.

Diseased or damaged blood vessels, such as those having aneurysms and/or dissections, can be non-invasively treated with endoluminal prosthetic devices or endografts that preserve blood flow through the damaged blood vessel. Many vascular aneurysms, dissections or other tissue defects occur in the aorta and peripheral arteries, and minimally invasive surgical techniques have been developed to place occlusive devices within or across an opening or cavity associated with the subject tissue defect to prevent blood from further pressurizing the damaged vascular tissue.

Conventional endograft devices can span the diseased region and effectively seal off the opening or cavity from the remaining healthy or intact blood vessel. In the instances of treating aortic aneurysms (e.g., abdominal aortic aneurysms, thoracic aorta aneurysms), the aneurysmal region of the aorta can be bypassed by use of an endoluminally delivered tubular exclusion device, such as a stent-graft, placed inside the vessel and spanning the aneurysmal portion of the aorta to seal off the aneurysmal portion from further exposure to blood flowing through the aorta. Stent-grafts, which are usually metal stents that are covered or lined by a graft or sealing material, can be delivered transluminally (e.g., introduced through the femoral artery) and implanted using specialized delivery catheters. Such endograft devices typically have a radially-compressed configuration or profile suitable for delivery through small-diameter guide catheters positionable within the aorta and branch vessels thereof. Percutaneous, transcatheter delivery of endograft devices to accommodate various vascular regions, as well as unique or otherwise diseased human anatomy, can be challenged by the delivery profiles of the devices in their radially-compressed states being too large. However, further reduction of the delivery profiles of the devices, and thereby the delivery catheters, can compromise radial strength of the endograft devices when deployed. Document US 2006/0149364 A1 describes a low profile vascular graft. WO 03/003945 A2 according to its Title relates to an implant having improved fixation to a bodily lumen.

### BRIEF SUMMARY OF THE INVENTION

Embodiments hereof are directed to endoluminal prosthetic devices for repair of vascular tissue defects, such as aortic aneurysms and/or dissections. In various arrangements, prosthetic devices for repairing a vascular tissue defect can be adjustable from a compressed configuration for delivery within a vasculature and a radially-expanded configuration for deployment within a target blood vessel in a patient. In an embodiment, a prosthesis includes a tubular body that can have a first end and a second end, wherein the first end can have an anchoring structure to engage an inner wall of the target blood vessel in the radially-expanded configuration. The tubular body also includes an elongated mid-portion between the first and second ends and which includes a channel formed in a wall thereof. The channel is at least partially oriented circumferentially about the tubular body. The prosthesis also includes a fluid-absorbable composition deposited within the channel. The fluid-absorbable composition can have a first volume when the prosthesis is in the compressed configuration and is configured to swell to a second volume within the channel upon deployment of the prosthesis within the target blood vessel to thereby transition at least the elongated mid-portion into the radially-expanded configuration. In some embodiments, one or more wires can be disposed within the channel and the fluid-absorbable composition can at least partially surround the wire.

In another embodiment, an expandable prosthetic device for implantation at a target blood vessel region to treat a target tissue defect in a patient can include a tubular body formed of a graft material. The tubular body can have a wall between first and second ends and a lumen defined by the wall. The device may also include a self-expanding anchor stent coupled to the first end for anchoring within the target blood vessel region when the device is implanted. The device may further include a plurality of expandable flanges arranged on an outer surface of the wall of the tubular body in a geometric pattern, wherein each expandable flange includes an encapsulation material coupled to the outer surface of the wall for forming a channel therebetween. Further, each expandable flange can include a fluid-absorbable composition contained within the channel, wherein the fluid-absorbable composition at least partially swells upon exposure to bodily fluids *in situ.* In this embodiment, at least partial swelling of the fluid-absorbable composition within the channel aids in radial expansion of the tubular body.

### BRIEF DESCRIPTION OF DRAWINGS

The foregoing and other features and aspects of the present technology can be better understood from the following description of embodiments and as illustrated in the accompanying drawings. The accompanying drawings, which are incorporated herein and form a part of the specification, further serve to illustrate the principles of the present technology. The components in the drawings are not necessarily to scale.
FIGS. 1A-1C are schematic illustrations of a healthy aorta, an abdominal aortic aneurysm and a thoracic aneurysm, respectively.
FIG. 2 is a side view of an endoluminal prosthesis in a radially-expanded configuration in accordance with an embodiment of the present technology.
FIG. 3A is a sectional view of the prosthesis taken along line 3A-3A of FIG. 2 and in accordance with an embodiment of the present technology.
FIG. 3B illustrates the prosthesis of FIG. 3A following exposure to fluid and in accordance with an embodiment of the present technology.
FIG. 3C is a sectional view of the prosthesis taken along line 3A-3A of FIG. 2 in accordance with another embodiment hereof.
FIG. 3D illustrates the prosthesis of the embodiment of FIG. 3C following exposure to fluid in accordance with another embodiment hereof.
FIG. 4A is a sectional view of the prosthesis taken along line 3A-3A of FIG. 2 and in accordance with another embodiment of the present technology.
FIG. 4B illustrates the prosthesis of FIG. 4A following exposure to fluid and in accordance with an embodiment of the present technology.
FIG. 5A is a sectional view of the prosthesis taken along line 3A-3A of FIG. 2 and in accordance with a further embodiment of the present technology.
FIG. 5B illustrates the prosthesis of FIG. 5A following exposure to fluid and in accordance with an embodiment of the present technology.
FIGS. 6A-6C are side views of various endoluminal prosthetic devices in accordance with additional embodiments of the present technology.
FIG. 7 illustrates a partial transparent view of an aorta displaying an abdominal aortic aneurysm and showing the prosthesis of FIG. 2 implanted within the aorta at a target vessel region in accordance with an embodiment of the present technology.
FIG. 8 is a side view of an endoluminal prosthesis in a radially-expanded configuration in accordance with another embodiment of the present technology.
FIG. 9 schematically shows a step of a method of delivering the prosthesis of FIG. 2 to a target site in the abdominal aorta in accordance with an embodiment of the present technology.

### DETAILED DESCRIPTION OF THE INVENTION

Specific embodiments of the present technology are now described with reference to the figures, wherein like reference numbers indicate identical or functionally similar elements. Unless otherwise indicated, the terms "distal" and "proximal" are used in the following description with respect to the direction of blood flow from the heart and through the vasculature. Accordingly, with respect to a prosthesis, the terms "proximal" and "distal" can refer to the location of portions of the device with respect to the direction of blood flow. For example, proximal can refer to an upstream position or a position of blood inflow, and distal can refer to a downstream position or a position of blood outflow. For example, "distal" or "distally" indicates an apparatus portion distant from, or a direction away from the heart or along the vasculature in the direction of blood flow. Likewise, "proximal" and "proximally" indicates an apparatus portion near to, or in a direction towards the heart.

The following detailed description is merely exemplary in nature and is not intended to limit the present technology or the application and uses of the present technology. Although the description of embodiments hereof are in the context of treatment of tissue defects in blood vessels, the present technology may also be used in any other body passageways or other blood vessel locations not specifically discussed herein and where it is deemed useful (e.g., other anatomical lumens, such as bronchial and other air passageways, fallopian tubes, bile ducts, etc.). Furthermore, there is no intention to be bound by any expressed or implied theory presented in the preceding technical field, background, brief summary or the following detailed description.

Embodiments of the present technology as described herein can be combined in many ways to treat one or more of many vascular defects such as aneurysms or dissections within a blood vessel, such as the abdominal or thoracic regions of the aorta. The embodiments of the present technology can be therapeutically combined with many known surgeries and procedures, for example, such embodiments can be combined with known methods of accessing the target tissue defects, such as percutaneous access of the abdominal or thoracic regions of the aorta through the femoral artery to deliver and deploy the endoluminal prosthetic devices described herein. Other routes of access to the target regions are also contemplated and are well known to one of ordinary skill in the art.

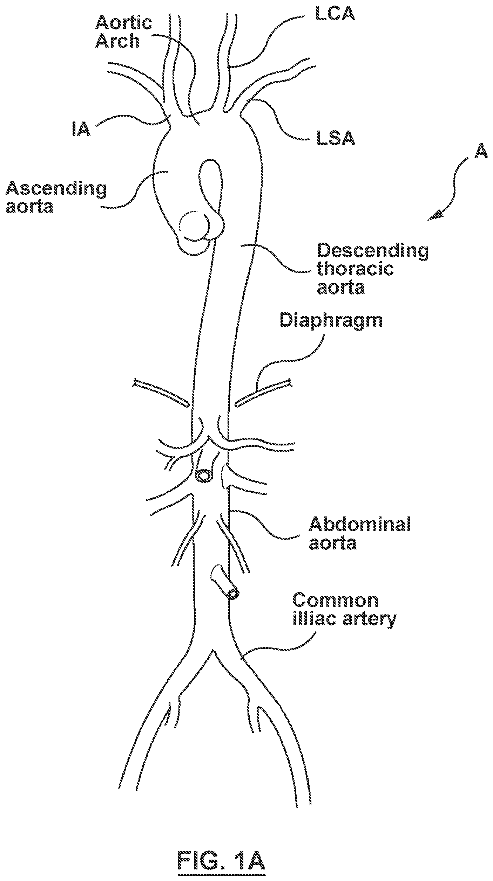

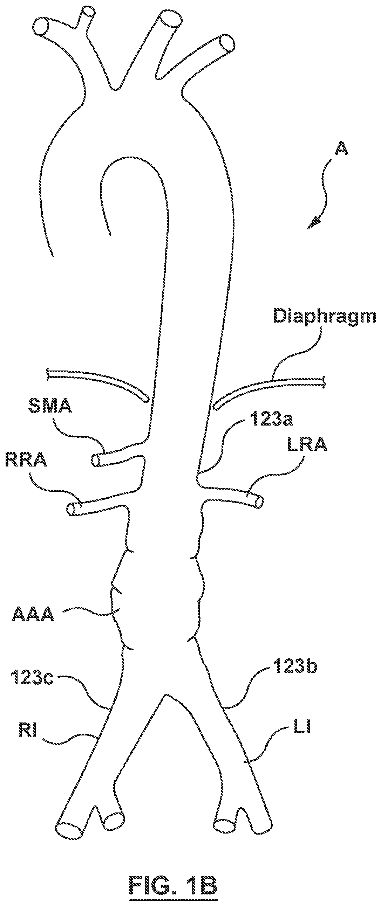

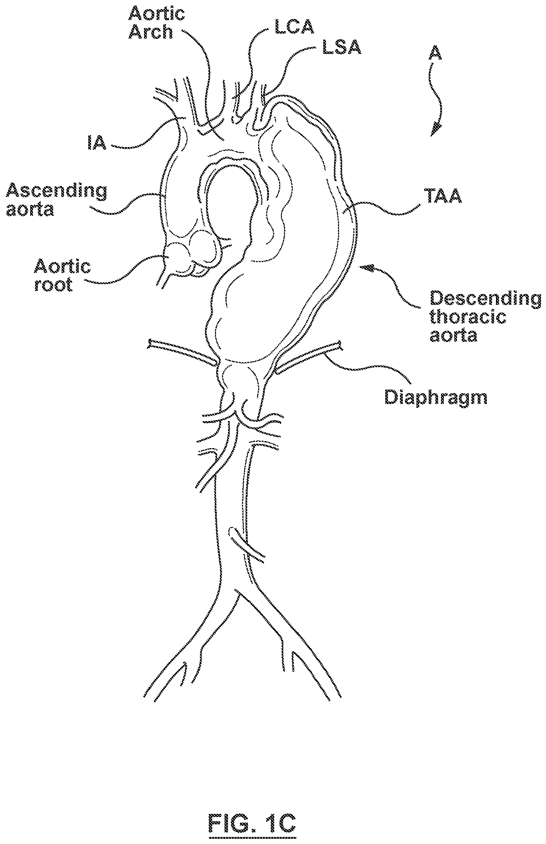

FIG. 1A illustrates a healthy human aorta A. The abdominal region of the aorta A is located distal to the diaphragm and the thoracic region of the aorta A is proximal to the diaphragm. As illustrated in FIG. 1B, abdominal aortic aneurysms (AAA) include aneurysms present in the aorta A distal to the diaphragm, e.g., pararenal aorta and the branch arteries that emanate therefrom, including the right and left renal arteries (RRA, LRA) and the superior mesenteric artery (SMA). As illustrated in FIG. 1C, thoracic aorta aneurysms (TAA) occur in the chest area and can involve the aortic root, ascending aorta, aortic arch or descending aorta. Aortic aneurysms are bulges or weakening regions in the aortic wall that can occur as a fusiform (e.g., uniform in shape) or as saccular (e.g., on one side of the aorta) aneurysms. As illustrated in FIG. 1B, the aorta A is shown extending down to the aortic bifurcation in which aorta A bifurcates into the common iliac arteries, including a right iliac artery RI and a left iliac artery LI. A right renal artery RRA and a left renal artery LRA extend from aorta A, as does the superior mesenteric artery (SMA) which arises from the anterior surface of the abdominal aorta. In certain instances, an abdominal aortic aneurysm AAA will affect regions including or adjacent to these branch arteries. Likewise, a thoracic aortic aneurysm TAA (FIG. 1C) can affect arteries branching from the aortic arch, such as the left subclavian artery (LSA), the left common carotid artery (LCA) and the innominate artery (IA).

As discussed herein, the aneurysmal region of the aorta can be bypassed by use of an endoluminally delivered tubular exclusion device, wherein proximal and distal ends of the device provide an occlusive seal when in contact with healthy portions of the vessel. The aforesaid challenges include providing a low profile during percutaneously delivery of the device while also providing a suitable structure having sufficient radial support once deployed to secure the device in position, providing a sealing affect against the wall of the vessel to prevent blood leakage into the tissue defect region, and providing a blood flow path through the internal lumen of the device.

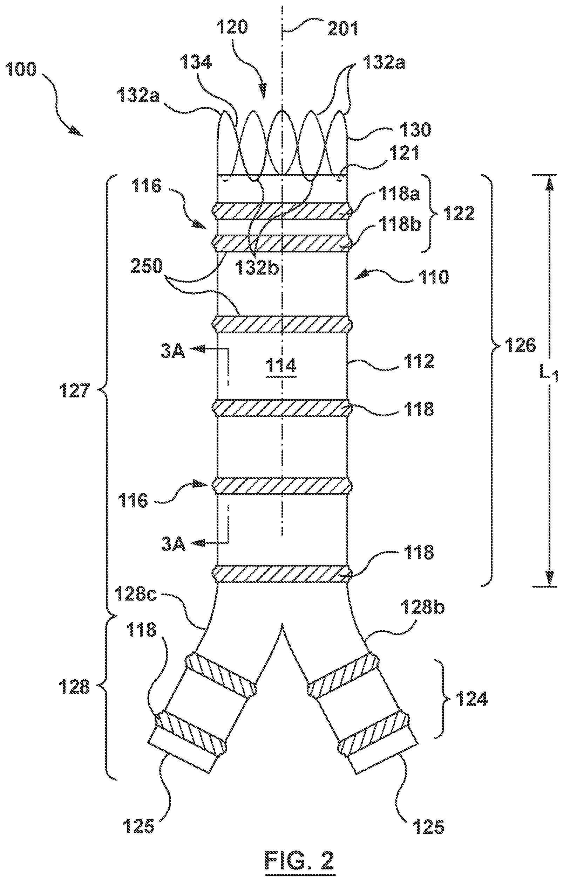

Embodiments of endoluminal prosthetic devices in accordance with the present technology are described in this section with reference to FIGS. 2-9. It will be appreciated that specific elements, substructures, uses, advantages, and/or other aspects of the embodiments described herein and with reference to FIGS. 2-9 can be suitably interchanged, substituted or otherwise configured with one another in accordance with additional embodiments of the present technology.

### Selected Embodiments of Endoluminal Prosthetic Devices

Provided herein are systems, devices and methods suitable for delivery and implantation of endoluminal prosthetic devices in a blood vessel of a patient. In some embodiments, methods and devices are presented for the treatment of vascular diseases, such as aneurysms and dissections, by minimally invasive implantation of artificial or prosthetic devices. For example, an endoluminal prosthetic device, in accordance with embodiments described herein, can be implanted for repair (e.g., occlusion) of a diseased or damaged segment of the aorta in a patient, such as in a patient suffering from an abdominal aortic aneurysm AAA illustrated in FIG. 1B or a thoracic aortic aneurysm TAA illustrated in FIG. 1C. In further embodiments, the prosthetic device is suitable for implantation and repair (e.g., occlusion) of other diseased or damaged blood vessels or other suitable anatomical lumens. FIG. 2 is a side view of an endoluminal prosthetic device or prosthesis 100 suitable for repair of a tissue defect in the abdominal aorta, such as an abdominal aortic aneurysm AAA (shown in FIG. 1B) in accordance with an embodiment of the present technology

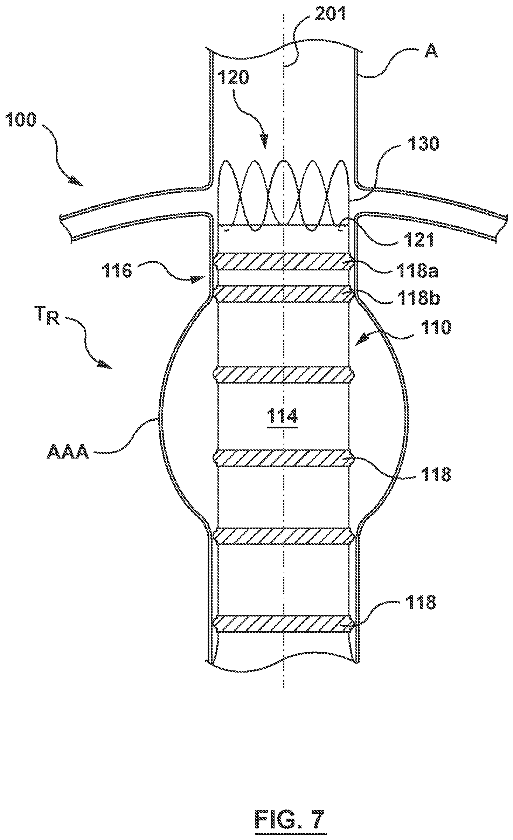

The prosthesis 100 can be movable between a radially-contracted (e.g., delivery) configuration (not shown), a radially-expanded configuration (FIG. 2), and a deployed configuration (discussed further below with respect to FIG. 7). In the radially-contracted configuration, the prosthesis 100 has a low-profile suitable for delivery through small-diameter guide catheters positionable within the aorta and branch vessels thereof via approach through, for example, the femoral artery. As used herein, "radially-expanded configuration" refers to the configuration of the device/assembly when allowed to freely expand to an unrestrained size without the presence of constraining or distorting forces. "Deployed configuration," as used herein, refers to the device/assembly once expanded at the target vessel site and subject to the constraining and distorting forces exerted by the native anatomy of the vessels and/or the other prosthesis components (if present).

With reference to FIG. 2, the prosthesis 100 may be an endograft prosthesis that is configured for placement in a main vessel, such as the abdominal aorta A. In certain embodiments, the prosthesis 100 is not a device custom designed for a particular patient's anatomy, but instead may be configured to treat infrarenal (i.e., located distal to the renal arteries), juxtarenal (i.e., approaches or extends up to, but does not involve, the renal arteries), and/or suprarenal (i.e., involves and extends above the renal arteries) aneurysms in a wide range of patient anatomies. As shown in FIG. 2, the prosthesis 100 includes an expandable tubular body 110 having a wall 112 formed of a graft or flexible material 114 and which is configured to transition between a low-profile (e.g., compressed) configuration suitable for delivery to the radially-expanded configuration. The prosthesis 100 transitions from the low-profile configuration to the radially-expanded configuration *in situ* with aid from an integrated structural scaffold 116. The structural scaffold 116 is provided by a plurality of enclosed channels 118 associated with the wall 112 of the tubular body 110 and a fluid-absorbable composition (not shown) deposited within the channels 118. The plurality of channels 118 are at least partially oriented circumferentially about the tubular body 110, such that when deployed *in situ,* together, the graft material 112 and the integrated structural scaffold 116, structurally define a lumen 120 of the prosthesis 100 through which blood can flow.

As shown in FIG. 2, the tubular body 110 has a generally tubular or cylindrical shape supported by the structural scaffold 116 and that further defines the lumen 120. The tubular body 110 has a first end 121 at a proximal segment 122, which may can define a proximal seal zone with a healthy portion 123a of the main vessel (FIG. 1B), and a pair of second ends 125 at distal segments 124, which define distal seal zones with healthy portions 123b, 123c (FIG. 1B) of the left iliac artery LI and the right iliac artery RI distal to the tissue defect to be occluded. Accordingly, the first and second ends 121, 125 are sufficiently spaced apart longitudinally such that an elongated mid-portion 126, having an appropriate longitudinal length Li, aligns with and seals off the target tissue defect (e.g., the opening or cavity of the aneurysm AAAs) from the healthy portions of the main vessel. When the prosthesis 100 is deployed, the elongated mid-portion 126 of the tubular body 110 substantially covers the opening or cavity created by the aneurysm AAA (FIG. 1B) and provides the proximal and distal seal zones with healthy portions 123a, 123b, 123c of the vessel, thereby excluding the defect tissue portion from blood flow through the vessel.

The tubular body 110 is generally defined by the graft material 114, and is shown having a main trunk segment 127 and a distal bifurcated segment 128 suitable to repair an abdominal aortic tissue defect. In an embodiment, the bifurcated segment 128 is integrally formed with trunk segment 127 as a single or unitary prosthesis 100. In another arrangement, the bifurcated segment 128 may be formed separately from the trunk segment 127 and coupled thereto, or in other embodiments, may not be a present feature of the tubular body 110. When deployed *in situ,* the trunk segment 127 is configured for placement within the abdominal aorta A and the bifurcated segment 128 having left and right legs 128b, 128c is configured for placement at the aortic bifurcation such that the left and right legs 128b, 128c thereof extend within the left and right common iliac arteries (LI, RI; FIG. 1B), respectively.

The tubular body 110 of the prosthesis 100 may be formed from one or more suitable graft or sealing materials 114, for example and not limited to, a woven or knit polyester, polytetrafluoroethylene (PTFE), expanded polytetrafluoroethylene (ePTFE), polyurethane, ultra-high-molecular-weight polyethylene (UHMWPE), or other suitable materials, such as polyethylene terephthalate (DACRON^{®} material), silicone or the like. In another embodiment, the graft material could also be a natural material such as pericardium or another membranous tissue such as intestinal submucosa.

The prosthesis 100 includes an anchor stent 130 coupled to the tubular body 110 at the first end 121. Optionally, anchor stents similar to the anchor stent 130 may be coupled at respective openings of the left and right legs 128b, 128c of the bifurcated segment 128 of the tubular body 110 to achieve acute seal/fixation with the vessels within which each is deployed, in order to provide fixation after initial deployment while the fluid-absorbable material is activated within channels 118. In one embodiment, the anchor stent 130 is a radially-compressible ring or scaffold that is operable to self-expand into apposition with an interior wall of a body vessel (not shown). As shown in FIG. 2, the anchor stent 130 is constructed from a self-expanding or spring material, such as nitinol, and is a sinusoidal patterned ring including a plurality of crowns or bends 132a, 132b and a plurality of struts or straight segments 134 with each crown being formed between a pair of opposing struts. In one embodiment, the anchor stent 130 is a laser-cut stent and the resulting bends 132a, 132b and struts 134 have a rectangular cross-section or approximately a rectangular cross-section. In another embodiment, the anchor stent 130 may be formed from a single, continuous wire that may be solid or hollow and have a circular cross-section. In still another embodiment, the cross-section of the wire that forms the anchor stent 130 may be an oval, square, rectangular, or any other suitable shape.

The anchor stent 130 can be coupled to the graft material 114 so as to have a first or proximal-most set of crowns 132a that extend outside of or beyond the first end 121 of the tubular body 110 in an open or exposed configuration and a second or opposing set of crowns 132b that is coupled to the first end 121 of the tubular body 110. The second set of crowns 132b can be coupled to the tubular body 110 by stitches, staples or other means known to those of skill in the art. In the embodiment shown in FIG. 2, the second set of crowns 132b are coupled to an outside surface of the tubular body 110. However, the crowns 132b may alternatively be coupled to an inside surface of the tubular body 110. The unattached first set of crowns 132a may include barbs (not shown) or other features for embedding into and anchoring into vascular tissue when the prosthesis 100 is deployed *in situ.*

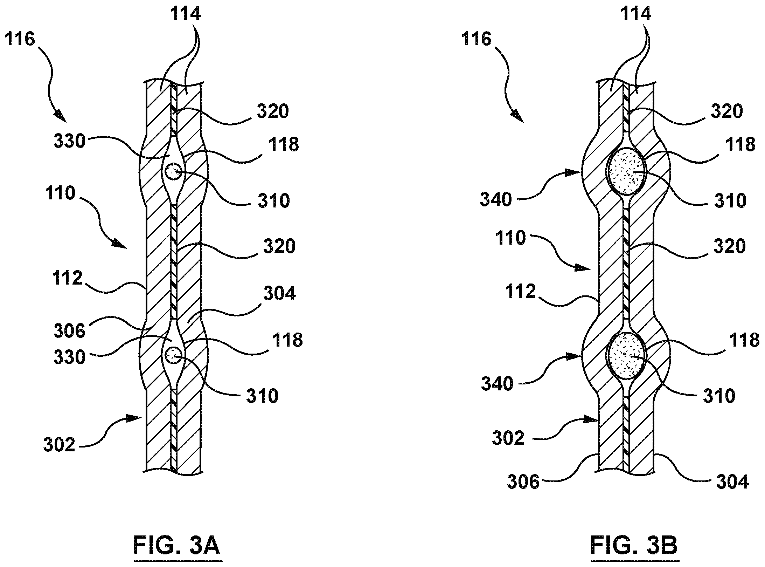

In accordance with embodiments hereof when implanting the prosthesis 100, the structural scaffold 116 is deployed by way of fluid permeation into the channels 118 to interact with the fluid-absorbable composition enclosed therein. FIGS. 3A and 3B are sectional views of the prosthesis 100 taken along line 3A-3A of FIG. 2 and showing the integrated structural scaffold 116 prior to (FIG. 3A) and after (FIG. 3B) fluid absorption by the fluid-absorbable composition deposited within the channels 118. The structural scaffold 116 is at least in part provided by channels 118 formed within the wall 112 of the tubular body 110. In this embodiment, the tubular body 110 includes a flexible sheet 302 of graft material 114 having opposing inner and outer layers 304, 306 that form the wall 112 and between which the channel 118 is defined. In one embodiment, the inner and outer layers 304, 306 are selected from different materials (e.g., PTFE, ePTFE, UHMWPE, polyurethane, woven polyester, etc.), however, in other arrangements, the materials may be the same. For example, in some embodiments the flexible sheet 302 can be a folded flexible material that forms the opposing inner and outer layers 304, 306 of the wall 112. At least one of the inner and outer layers 304, 306 is formed of a permeable or semi-permeable material that permits fluid, such as blood/plasma/serous fluid, to permeate into the enclosed channel 118 to interact with the fluid-absorbable composition 310 deposited and retained therein. In particular, the permeable or semi-permeable material allows for blood to flow into the enclosed channels 118 while excluding the fluid-absorbable composition 310 from flowing or leaking out of the channels 118.

In the embodiment shown in FIG. 3A, the channel 118 can be formed (e.g., via stitching, tape, staples, adhesive, heat bonding or other securing means) between the inner and outer layers 304, 306 and thereby provide the channel 118 into which the fluid-absorbable composition 310 can be deposited (e.g., injected, deposited by inserted tube or strand, etc.). In another embodiment, the fluid-absorbable composition 310 may be formed, such as a sheet, strip, strand, ribbon, thread, or other elongate shape, so as to be deposited along pre-determined portions of the inner and outer layers 304, 306 prior to securing and/or bonding the inner and outer layers together to form the flexible sheet 302. In a particular example, the graft material 114 can be selected from ePTFE, PTFE, UHMWPE and/or polyurethane, and the inner and outer layers 304, 306 can be laminated in zones 320. Lamination zones 320 can be created with heat and pressure applied at dies (not shown) that, during such manufacturing steps, are spaced apart from pre-deposited strips or strands of fluid-absorbable composition 310 to provide channels 118 having a larger cross-sectional dimension than the cross-sectional dimension of the fluid-absorbable composition 310 when deposited. The space 330 created by the larger cross-sectional dimension of the channels 118 allows for expansion of the fluid-absorbable composition 310 from a first volume (FIG. 3A) to a second volume (FIG. 3B) when exposed to fluid (e.g., blood). The cross-sectional dimension of the channel 118 can prevent swelling of the fluid-absorbable composition 310 beyond a volume that is accommodated by the channel 118 (FIG. 3B).

In operation, and upon swelling of the fluid-absorbable composition 310 to the second volume, hydrostatic pressure (e.g., the pressure exerted by the fluid-absorbing composition 310 as a result of its potential energy held within the confines of the enclosed channels 118) is generated, thereby creating turgid tubes or support structures 340 (FIG. 3B). In reference to FIGS. 2-3B, the plurality of turgid support structures 340 along the tubular body 110 together form the structural scaffold 116 of the prosthesis 100. At least partial swelling of the turgid support structures 340 causes radial expansion of the tubular body 110 thereby supporting the lumen 120 of the prosthesis 100 in an open or radially-expanded configuration *in situ* (FIG. 2) to permit blood flow therethrough.

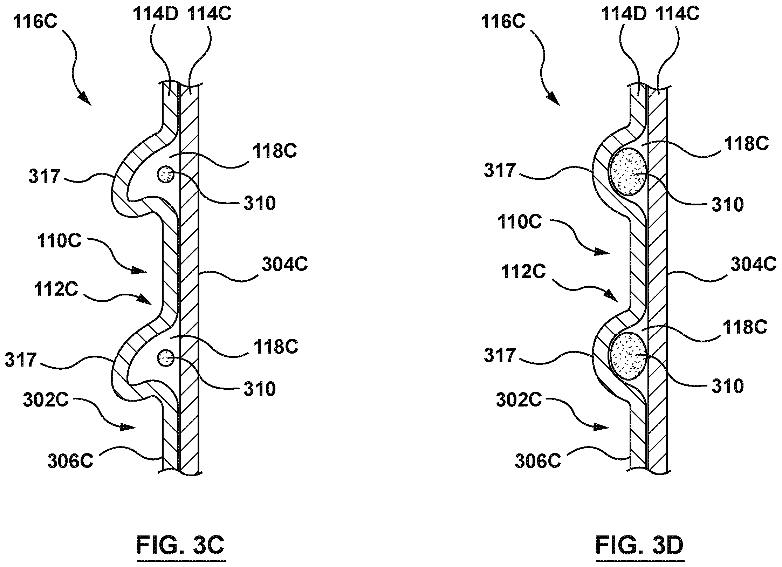

FIGS. 3C and 3D are alternate sectional views of the prosthesis 100 taken along line 3A-3A of FIG. 2 and showing a structural scaffold 116C prior to (FIG. 3C) and after (FIG. 3D) fluid absorption by a fluid-absorbable composition 310 deposited within channels 118C thereof. The structural scaffold 116C is at least in part provided by channels 118C formed within a wall 112C of the tubular body 110C. In this embodiment, the tubular body 110C is formed from a flexible sheet 302C having an inner layer 304C of a first material 114C and an opposing outer layer 306C of a second material 114D (which together form the wall 112C). The inner and outer layers 304C, 306C may be secured and/or bonded to each other to form the flexible sheet 112C in any suitable manner and/or as described above for the inner and outer layers 304, 306. In the embodiment of FIGS. 3C and 3D, each channel 118C is defined by a respective fold or flaps 317 in the second material 114D that forms the outer layer 306C with of the second material 114D being of an inelastic, impermeable nature. The first material 114C of the inner layer 304C is of a permeable or semi-permeable nature that permits fluid, such as blood/plasma/serous fluid, to permeate into the enclosed channel 118C so as to permit interaction with the fluid-absorbable composition 310 deposited and retained therein. In operation, swelling of the fluid-absorbable composition 310 fills the channel 118c and expands the flap 317 in the outer layer 306C toward a vessel side of the prosthesis 100 with little to no expansion of the inner layer 304C toward a lumenal side of the prosthesis. In this manner, the structural scaffold 116C produces minimal impingement of a lumen defined by tubular body 110C. As well the asymmetric weld design of the wall 112C would reduce foreshortening of the tubular body 110C when in the expanded configuration shown in FIG. 3D. In all other manner, the embodiment of FIGS. 3C and 3D functions similarly to the embodiment depicted and described with reference to FIGS. 3A and 3B.

Individual turgid support structures 340 (FIG. 3B) can be configured to be selectively flexible to deform as necessary to accommodate anatomical structures during implantation and at the target vessel region of implantation. The structural scaffold 116 comprised of the plurality of turgid support structures 340 may further provide an outward radial strength or buckling resistance to pressures that can be exerted on an outer surface of the wall 112 of the tubular body 110 following implantation, and such buckling resistance can prevent the lumen 120 from collapsing and/or otherwise from inhibiting blood flow through the lumen 120.

In embodiment in accordance herewith, the fluid-absorbable composition 310 can be a suitable hydrophilic and covalently cross-linked composition such as a natural or synthetic hydrophilic polymeric material capable of absorbing suitable quantities of water or other fluid (e.g., blood). In some embodiments, the fluid-absorbable composition 310 can be a hydrogel composition or, in other arrangements, a hydrophilic foam. A hydrogel is a polymer gel constructed of one or more networks of crosslinked hydrophilic polymer chains that can absorb large amounts (compared to its dry weight) of water via hydrogen bonding. Hydrogel compositions, in some instances, are capable of absorbing water (or other fluid) relative to its dry weight to greater than 50%, greater than 75%, greater than 100%, greater than 150%, etc. of its dry weight. In other embodiments, the hydrogel may be fully hydrated when containing less than 50% of its dry weight (e.g., less than 45%, less than 40%, etc.). In a dehydrated or low volume state, a hydrogel can, in some instances, be fairly rigid; however with certain compositions, the hydrogel can exhibit increased flexibility as water content increases, thereby allowing, for example, the hydrogel composition in its swollen or turgid state to radially-extend the tubular body 110 into the tubular or cylindrical shape (FIG. 2). Suitable hydrogel or other fluid-absorbable compositions 310 can be firm upon water absorption while maintaining elastic-mechanical properties (e.g., elastically or reversibly and temporarily distorting shape when force is applied).

One or more hydrophilic polymeric materials can be selected for providing a fluid-absorbable composition 310. For example, the fluid-absorbable composition 310 may include a variety of hydrogel polymers, or other appropriate hydrophilic or hydrophobic materials, as well as other suitable materials, such as foams, interpenetrating polymer networks and thermosets. Such materials are described as examples, and these and other materials will be apparent to those of ordinary skill in the art. Accordingly, the present technology is not limited by the specific materials set forth herein. Synthetic materials capable of forming suitable hydrogels include polyethylene oxide, polyvinyl alcohol, polyacrylic acid, polypropylene fumarate-co-ethylene glycol, and polypeptides. Agarose, alginate, chitosan, collagen, fibrin, gelatin, and hyaluronic acid are naturally-derived polymers that could also be used for this purpose. For example, illustrative polymers suitable for incorporation within the channels 118, can include poly-2-hydroxyethylmethacrylate (p-HEMA) and copolymers thereof, poly-N-vinyl-pyrrolidone (pNVP) hydrogels, pHEMA/pNVP copolymer, polyvinylalcohol (PVA) hydrogels, and other similar materials. In particular embodiments, the polymeric materials are biocompatible and biostable.

Advantageously, the fluid-absorbable composition 310 can be deposited within the channel in a strip or strand that has a first volume on the order of microns thick (e.g., about 100-500 µm, 300-700 µm, 500-900 µm, etc.) but will swell upon exposure fluid (e.g., blood) to have a second cross-sectional dimension up to approximately 1000 times the first cross-sectional dimension. Accordingly, the prosthesis 100 can have a reduced or lower delivery profile when in a radially contracted configuration than a delivery profile of a conventional stent-graft having stent structures comprising self-expanding or balloon-expandable struts.

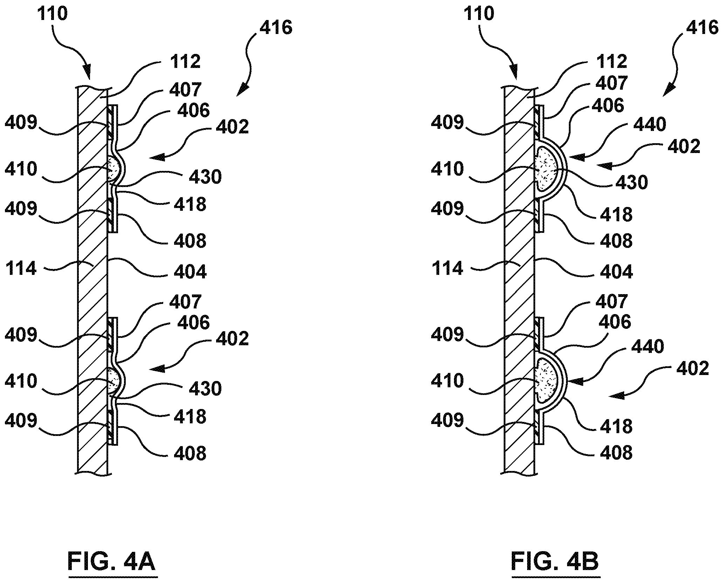

FIGS. 4A and 4B illustrate the prosthesis 100 taken along lines 3A-3A of FIG. 2 in accordance with another embodiment and showing an integrated structural scaffold 416 prior to (FIG. 4A) and after (FIG. 4B) fluid absorption by a fluid-absorbable composition 410. Structural scaffold 416 includes a plurality of expandable flanges 402 arranged on an outer surface 404 of the wall 112 of the tubular body 110. As illustrated in FIG. 4A, each expandable flange 402 includes an encapsulation material 406 coupled to the outer surface 404 of the wall 112 on first and second sides 407, 408 for forming an enclosed channel 418 between the outer surface 404 of the wall 112 and the encapsulation material 406. Each flange 402 further includes a fluid-absorbable composition 410 (e.g., fluid-absorbable compositions 310 described above such as a hydrogel) contained within the channel 418. Operatively, fluid absorption by the fluid-absorbable composition 410 from the first volume (FIG. 4A) to a second volume (FIG. 4B) causes expansion of the expandable flanges 402 on the outer surface 404 of the wall 112 of the tubular body 110. At least partial expansion of the flanges 402 *in situ* can aid in radial expansion of the tubular body 110 and maintain the lumen 120 of the prosthesis 100 in an open position for accommodating blood flow therethrough (FIG. 2).

In the embodiment illustrated in FIGS. 4A and 4B, the graft material 114 forming the wall 112 of the tubular body 110 can be polyethylene (e.g., UHMWPE), or in another embodiment, polyethylene terephthalate (DACRON^{®} material). The encapsulation material 406 can be formed of, for example, polyurethane, ePTFE polyethylene (e.g., UHMWPE), or polyester. In alternative arrangements, the graft material 114 can be a non-porous graft material, such as ePTFE and fluid access points (not shown) to the channel 418 can be provided to allow fluid penetration *in situ* and subsequent swelling of the fluid-absorbable composition 410 enclosed therein. In additional embodiments, the encapsulation material 406 can be a non-porous material (e.g., ePTFE) and the graft material 114 can provide a porous layer (e.g., woven polyester). In certain embodiments, a polyester layer can provide suitable permeation of fluid *in situ* for a first period of time (e.g., the fluid-absorbable composition can swell to 85%-95% of the second volume within approximately 20 minutes), but becomes impermeable over time to facilitate sealing and occlusion of the target tissue defect.

Coupling of first and second sides 407, 408 of the encapsulation material 406 to the graft material 114 on the outer surface 404 of the wall 112 can be accomplished via heat welding/bonding at bonding zones 409, for example, which run along opposing edges of first and second sides 407, 408 of the encapsulation material 406. Other methods (e.g., stitching, tape, staples, adhesive or other securing means) of attaching the encapsulation material 406 to the graft material 114 of the wall 112 are also known to those of ordinary skill in the art. The first and second sides 407, 408 of the encapsulation material 406 are coupled to the wall 112 in a manner that defines tubes (e.g., an enclosed compartment) or channel 418 between the wall 112 and the encapsulation material 406, which in an unexpanded state may loosely lay like folds 317 described above and shown in FIG. 3C. Contained within the channels 418 is the fluid-absorbable composition 410 having a first (e.g., non-swollen) volume (FIG. 4A). In one embodiment, the fluid-absorbable composition 410 is deposited on the outer surface 404 of the wall 112 and the encapsulation material 406 is coupled to the outer surface 404 while spanning the fluid-absorbable composition 410 to form the enclosed channel 418.

FIG. 4B illustrates the prosthesis of FIG. 4A following exposure to fluid and in reference to FIGS. 4A and 4B together, the channels 418 are sized and configured to include a space 430 for accommodating swelling of the fluid-absorbable composition 410 when exposed to fluid (e.g., following implantation). For example, the channels 418 (e.g., tubes defined on the tubular body 110 by the coupled encapsulation material 406) are configured to limit the swelling of the fluid-absorbable composition therein. During deployment, the fluid-absorbable composition 410 absorbs blood *in situ* and at least partially swells to a second volume within the confines of the enclosed channels 418 and generates hydrostatic pressure therein. Each of the pressurized channels 418 creates a separate turgid tube or support structure 440 (FIG. 4B) on the outer surface 404 of the wall 112, providing the expansion of the expandable flanges 402 on the outer surface 404 and aid in radial expansion of the tubular body 110 (FIG. 2) as well as provide an outward radial strength to the wall 112. Additionally, in some arrangements, the expansion of the expandable flanges 402 on the outer surface 404 of the wall 112 provide a radial force against an inner wall of the target blood vessel region for anchoring the prosthesis 100 and/or assisting in sealing the prosthesis 100 against the vessel.

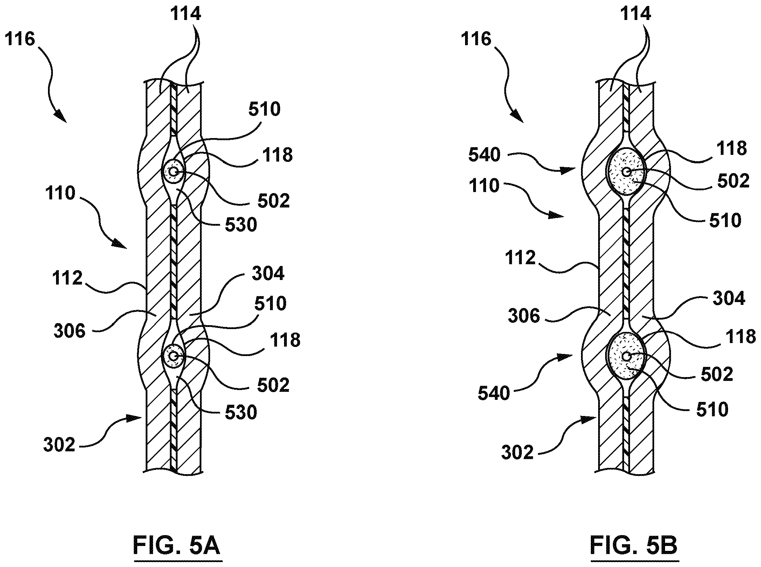

FIGS. 5A and 5B illustrate yet another embodiment of the prosthesis 100 taken along lines 3A-3A of FIG. 2. The embodiment shown in FIGS. 5A and 5B include many similar features as the embodiment shown in FIGS. 3A and 3B. For example, the embodiment illustrated in FIGS. 5A and 5B include the tubular body 110 having a flexible sheet 302 of graft material 114 with opposing inner and outer layers 304, 306 that form the wall 112 and between which the channel 118 is defined. However, in the embodiment shown in FIGS. 5A and 5B, the prosthesis 100 further includes one or more wires 502 disposed within one or more of the enclosed channels 118. As illustrated in FIG. 5A, a fluid-absorbable composition 510 can at least partially surround the wire 502 disposed within each channel 118. In one embodiment, the fluid-absorbable composition 510 can be provided as a coating on the wire 502 prior to disposing the wire within the channel 118.

In one embodiment, the wires 502 can transition between a radially-compressed configuration suitable for delivery in a low-profile delivery catheter and a radially-expanded configuration (FIG. 2). The wires 502 can be provided as rings or other expandable features that can be self-expanding and/or balloon expandable as is known in the art. The term "self-expanding" is used to convey that the structures are shaped or formed from a material that can be provided with a mechanical memory to return the structure from a radially-compressed or constricted delivery configuration to a radially-expanded configuration for deployment. Non-exhaustive exemplary self-expanding materials include stainless steel, a super-elastic metal such as a nickel titanium alloy or nitinol, various polymers, or a so-called super alloy, which may have a base metal of nickel, cobalt, chromium, or other metal. Mechanical memory may be imparted to a wire or other stent structure, such as the anchor stent 130, by thermal treatment to achieve a spring temper in stainless steel, for example, or to set a shape memory in a susceptible metal alloy, such as nitinol. Various polymers that can be made to have shape memory characteristics may also be suitable for use in embodiments hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

In the embodiment illustrated in FIG. 5A, the wire 502 can have a reduced or minimal thickness, for example, 0.001 inch to 0.010 inch, when compared to a thickness of a typical or conventional wire, for example, 0.013 inch, used in stent structures for radially-expanding and providing support to conventional stent-graft prosthetic devices. Accordingly, the wires 502 will have a reduced or lower profile than the typical or conventional wires of known stent structures. In the presently illustrated embodiment, the reduced profile wire 502 is coated and/or at least partially surrounded by the fluid-absorbable composition 510 which, upon exposure to fluid (e.g., blood), swells to fill space 530 within the channel 118, thereby providing turgid support structures 540 that are further strengthened against buckling and/or are provided with further elastic mechanical properties by the wires 502 disposed within (FIG. 5B). In one arrangement, the channels 118 provided along the tubular body 110 (FIG. 2) may each include a wire 502 coated with the fluid-absorbable composition 510 disposed therein; however, in alternative arrangements, only some of the channels 118 may include a wire 502 coated with the fluid-absorbable composition 510. In still other arrangements, the tubular body 110 may incorporate wires or other stent-like structures coupled to the graft material 114 in regions other than within the channels 118.

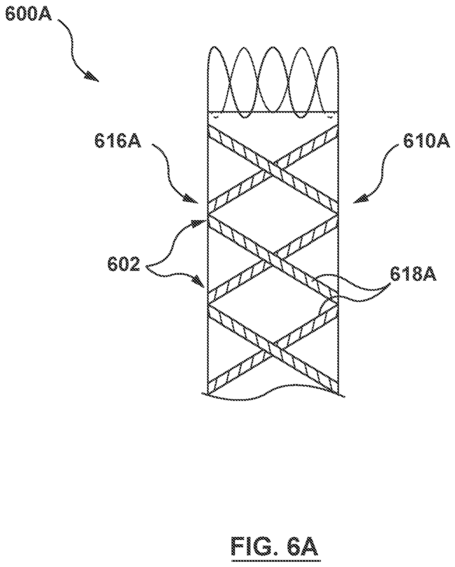

Referring back to FIG. 2, the plurality of channels 118 are at least partially oriented circumferentially about the tubular body 110 to provide the integrated structural scaffold 116 with a plurality of circumferential rings 250 spaced apart longitudinally along a longitudinal axis 201 of the tubular body 110. In other embodiments in accordance herewith, the integrated structural scaffold 116 can also be provided in other geometric patterns on the wall 112 of the tubular body 110. For example, FIGS. 6A-6C are side views of endoluminal prosthesis 600A, 600B, 600C showing additional geometric patterns provided by integrated structural scaffolds 616A, 616B, 616C in accordance with additional embodiments of the present technology. In particular, FIGS. 6A-6C illustrate a variety of configurations in which channels 618A, 618B, 618C may be formed on the tubular body 110.

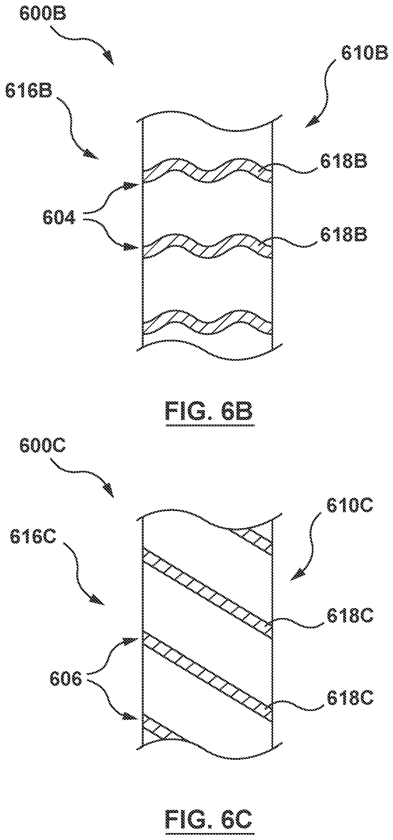

FIG. 6A, for example, illustrates an embodiment of a tubular body 610A having channels 618A provided in a crisscross pattern 602. FIGS. 6B and 6C are partial views of a tubular body 610B, 610C of endoluminal prosthesis 600B, 600C, respectively. Tubular body 610B includes channels 618B each of which has a sinusoidal pattern 604 as it encircles the tubular body 610B. Tubular body 110 610C includes channels 618C arranged in a spiral pattern 606 thereabout. In other embodiments, enclosed channels in accordance herewith may be formed in a diamond pattern or a chevron pattern (not shown). In still other embodiments, a tubular body may have enclosed channels provided in a combination of geometric patterns there along thereof.

In still further embodiments, and with reference to FIG. 2, the integrated structural support 116 can include regions (e.g., near first and/or second ends 121, 125) of the tubular body 110 having a higher density of fluid-absorbing composition filled channels 118 for increasing radial strength, buckling resistance and/or outward compressive force and the like. For example, the spacing between channels 118a and 118b proximate to the first end 121 is narrower than spacing between remaining channels 118 formed along the mid-portion 126. In other embodiments, as illustrated in FIG. 6A, the arrangement of channels 618A of the integrated structural support 616A may be approximately uniform along the entire length thereof.

While some endograft devices can span the diseased region and effectively seal off the opening or cavity from the remaining healthy or intact blood vessel, challenges arise when the diseased regions are in the vicinity of vessel bifurcations or "branch" vessels that continue to require patent blood flow to maintain other tissues or organs. For example, depending on the region of the aorta involved, an aneurysm may extend into segments of the aorta from which smaller branch arteries extend. Various arrangements have been proposed and implemented to accommodate side branches, including deployment of branch stent assemblies in parallel with the main prosthesis (e.g., prosthesis 100). When deployed together, the branch stent assemblies can direct blood from the main vessel, through the proximal seal zone and into the branch vessel using a "snorkel" or chimney technique for endovascular aortic aneurysm repair (chEVAR).

FIG. 7 illustrates a partial transparent view of an aorta A displaying a suprarenal abdominal aortic aneurysm AAA and showing the prosthesis 100 of FIG. 2 implanted within the aorta A at the target vessel region T_{R} in accordance with an embodiment of the present technology. As shown in FIG. 7 in the deployed configuration, channels 118a, 118b of the scaffold 116 that are disposed at the first end 121 of the tubular body portion 110 are expanded to be in apposition with the main vessel near the ostium of the branch vessels on a first end of the AAA with a channel 118c along the body portion 110 expanded to be in apposition with the main vessel on an opposite second end of the AAA. In the placement of the prosthesis 100 shown in FIG. 7, the anchor stent 130 extending from the first end 121 of the prosthesis 100 is implanted to span and to be in apposition with the wall of the aorta on each side of the ostium of the branch vessels. When initially placed within the vessel, the anchor stent 130 is configured to provide adequate radial strength/stability to the tubular body 110 to prevent the graft material 114 from invagination during activation of the fluid-absorbable composition 310 within the channels 108, wherein activation of the fluid-absorbable composition 310 within the channels 108 may take several minutes after implantation before the channels 108 attain sufficient radially strength to maintain the lumen 120 of the prosthesis 100. After activation of the fluid-absorbable composition 310 is complete, the anchor stent 130 in combination with the expanded structural scaffold 116 of the tubular body 110 are configured to provide a generally radially outward force that ensures the tubular body 110 is contacting and sealing against the wall of the main vessel (as described above) and that maintains the integrity of the lumen 120 of the prosthesis 100.

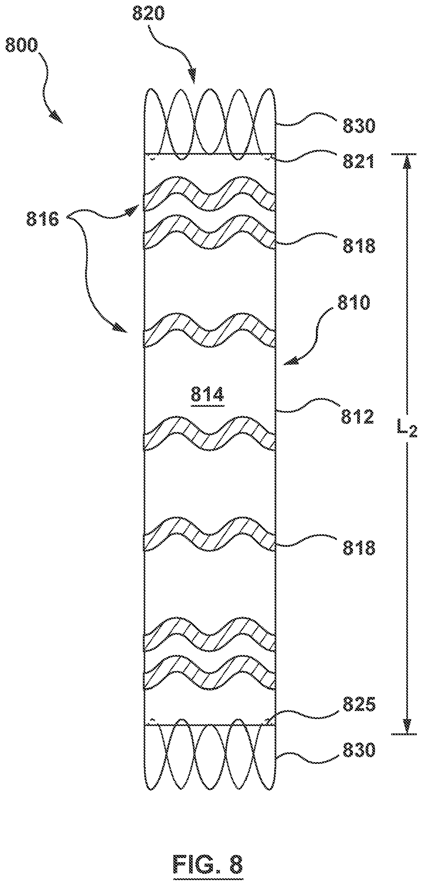

FIG. 8 is a side view of an endoluminal prosthesis 800 in a radially-expanded configuration in accordance with another embodiment of the present technology. With reference to FIG. 8, the prosthesis 800 can have similar features and characteristics as the prosthesis 100 of FIG. 2; however, the prosthesis 800 is configured for placement in a main vessel such as a portion of the descending aorta A for treating a thoracic aortic aneurysm TAA (FIG. 1C) and/or other main vessel regions not having a bifurcation. In particular, the prosthesis 800 includes an expandable tubular body 810 having a wall 812 formed of a graft material 814 and which is configured to transition between a low-profile (e.g., compressed) configuration suitable for delivery to the radially-expanded configuration (FIG. 8). In a similar manner as the prosthesis 100, the prosthesis 800 is configured to transition from the low-profile configuration to the radially-expanded configuration *in situ* with aid from an integrated structural scaffold 816. The structural scaffold 816 can be provided by a plurality of enclosed channels 818 associated with the wall 812 of the tubular body 810 and a fluid-absorbable composition (not shown) deposited within the channels 818 as described above. Furthermore, the plurality of channels 818 are at least partially oriented circumferentially about the tubular body 810 and can be provided in a variety of geometric patterns (e.g., longitudinally-spaced apart circumferential rings, a diamond pattern, a chevron pattern, a crisscross pattern, a spiral pattern, a sinusoidal pattern, or combination thereof, etc.) as described with respect to the prosthesis 100, 600A, 600B, 600C. When deployed at the target tissue defect region, the graft material 812 of the tubular body 810 and the integrated structural scaffold 816 structurally define a lumen 820 of the prosthesis 800 through which blood can flow. The prosthesis 800 can further include an anchor stent 830 coupled to the tubular body 810 at first and/or second ends 821, 825 capable of expanding into apposition with an interior wall of the vessel (not shown) at the target tissue region. In other arrangements, anchor stents 830 can be provided at one of the first or second ends 821, 825 or not present on the prosthesis 800.

As shown in FIG. 8, tubular body 810 has an appropriate longitudinal length L₂ to seal off the target tissue defect (e.g., the opening or cavity of the aneurysm) from the healthy portions of the main vessel. For example, when the prosthesis 800 is deployed, the tubular body 810 substantially covers the opening or cavity created by the aneurysm TAA (FIG. 1C) and excludes the defect tissue portion from blood flow through the vessel. The integrated structural scaffold 816 allows for flexibility of the prosthesis 800 to accommodate natural bends in the vasculature at the target tissue region, such as, for example, at or near the aortic arch (FIG. 1C).

### Selected Systems and Methods for Delivery and Implantation of Endoluminal Prosthetic Devices

Suitable delivery and deployment methods are discussed herein and discussed further below; however, one of ordinary skill in the art will recognize a plurality of methods suitable to deliver the prosthesis 100, 600A-600C or 800 to the target vessel region (e.g., percutaneous, transcatheter delivery, for example, using a femoral artery approach). Additionally, one of ordinary skill in the art will recognize a plurality of methods suitable to deploy the prosthesis 100 or 800 from a compressed configuration for delivery to the deployed configuration, or radially-expanded configuration *in situ.*

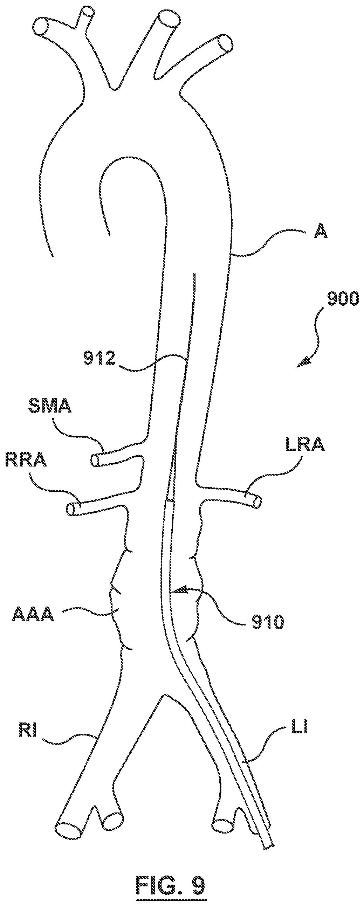

FIG. 9 shows a delivery system 900 for delivering and deploying the prosthesis 100 of FIG. 2 in the abdominal aorta for the repair of an abdominal aortic aneurysm AAA in accordance with an embodiment of the present technology. The delivery system 900 can include a delivery catheter 910 configured for delivery and deployment of the prosthesis 100, including the tubular body 110 and the integrated structural scaffold 116 (FIG. 2), radially-compressed therein. The delivery catheter 910 advances over a guidewire 912 and to the target vessel region in the abdominal aorta A. The guide wire 912 is typically inserted into the femoral artery (not shown) and percutaneously routed upstream through the left iliac artery LI to the abdominal aorta A, as is known in the art. Delivery of the delivery catheter 910 can also occur through right iliac artery RI. The location of the delivery catheter 910 and/or the prosthesis 100 may be verified radiographically when the delivery system 900 and/or prosthesis components include radiopaque markers, as is known in the art. For example, in one embodiment, the first and/or second ends 121, 125 of the tubular body 110 (FIG 2) may include radiopaque markers to aid in positioning. The prosthesis 100 is held within the delivery catheter 910 in a compressed or collapsed configuration for delivery thereof. In such a compressed configuration, the fluid-absorbable composition disposed within the channels 118 (FIG. 2) of the prosthesis 100 may be incidentally exposed to various fluid, e.g., saline and blood, during delivery; however any wetting of the prosthesis 100 in the compressed state should not result in noticeable expansion of the fluid-absorbable composition because of the compressed state of the channels within which it is disposed. Upon implantation the outer delivery sheath 916 is retracted to deploy the prosthesis 100 and to permit the expansion of the anchor stent 130, which fixes the first end 121 of the prosthesis at the treatment site and also routes blood flow within the lumen 120 of the tubular body 110 to return the tubular body 110 to its tubular shape. As well, blood flow directed within the lumen 120 of the tubular body 110 permits fluid permeation into the channels 118 and at least partial absorption of the fluid by the fluid-absorbable composition to effectuate radial expansion of the tubular body 110 *in situ.*

Some conventional endoluminal stent-grafts designed for repairing aneurysms and other tissue defects in vessels such as the aorta have challenges in reducing delivery profile of the devices to a desirable range to accommodate a patient's vasculature and/or to perform procedures with more comfort to the patient. In particular, conventional metal stents used with these stent-grafts have mechanical requirements for imparting radial strength to the endoluminal devices and are therefore limited as to how thin the metal stents can be manufactured. Other challenges to providing a low profile delivery configuration occur with radially compressing metal stents (e.g., nitinol stents) having stiffness requirements and other crimp strain constraints which can factor into loading the stent-grafts into increasingly smaller delivery catheters.

In contrast to the issues relating to using the conventional approaches, the present technology provides prosthetic devices having integrated structural scaffolds that include channels provided within or on a wall of the tubular bodies thereof. The channels contain a fluid-absorbable composition in a dehydrated or first volume which can be radially-contracted into a significantly reduced low profile state (as compared to the conventional metal-stent-graft prosthesis) and be accommodated within a low profile or smaller delivery catheter. Furthermore, the crimp strain constraints and stiffness of the conventional metal stents can be avoided or reduced significantly when loading the prosthesis 100 within the delivery catheter.

### Additional Embodiments

Features of the endoluminal prosthetic devices described above and illustrated in FIGS. 2-9 can be modified to form additional embodiments configured in accordance with the present technology. For example, the integrated support structure 116 of the prosthesis 100 shown in FIG. 2 can include a combination of channels 118 formed between inner and outer layers of the wall (as illustrated in FIG. 3A) and channels 418 formed between an outer surface of the wall and an encapsulation material coupled thereto (as illustrated in FIG. 4A) Similarly, the endoluminal prosthetic devices described above and illustrated in FIGS. 2 and 4A-B showing the flanges on the outer surface of the wall of the tubular body as having only a fluid-absorbable composition therein, may also have a wire (such as illustrated in FIG. 5A) or other structure disposed therein. Other various embodiments described herein may also be combined to provide further embodiments.

Various method steps described above for manufacturing and/or delivery and deployment of the prosthesis for repairing a target tissue defect in a blood vessel of a patient also can be interchanged to form additional embodiments of the present technology. For example, while the method steps described above are presented in a given order, alternative embodiments may perform steps in a different order.

While various embodiments have been described above, it should be understood that they have been presented only as illustrations and examples of the present technology, and not by way of limitation. Thus, the breadth and scope of the present technology should not be limited by any of the above-described embodiments, but should be defined only in accordance with the appended claims. It will also be understood that each feature of each embodiment discussed herein can be used in combination with the features of any other embodiment.

## Claims

1. A prosthesis (100, 600A, 600B, 600C, 800) having a compressed configuration for delivery within a vasculature and a radially-expanded configuration for deployment within a target blood vessel in a patient, the prosthesis comprising:
a tubular body (110, 110C, 610A, 610B, 610C, 810) having
a first end (121, 821) and a second end (125, 825), the first end (121, 821) having an anchoring structure to engage an inner wall of the target blood vessel in the radially-expanded configuration; and
an elongated mid-portion (126) between the first (121, 821) and second ends (125, 825) and including a channel (118, 118a, 118b, 118c, 418, 618A, 618B, 618C, 818) formed in a wall (112, 112C, 812) thereof, wherein the channel (118, 118a, 118b, 118c, 418, 618A, 618B, 618C, 818) is at least partially oriented circumferentially about the tubular body (110, 110C, 610A, 610B, 610C, 810); and
a fluid-absorbable composition (310, 410, 510) deposited within the channel (118, 118a, 118b, 118c, 418, 618A, 618B, 618C, 818), the fluid-absorbable composition (310, 410, 510) having a first volume when the prosthesis (100, 600A, 600B, 600C, 800) is in the compressed configuration and configured to swell to a second volume within the channel (118, 118a, 118b, 118c, 418, 618A, 618B, 618C, 818) upon deployment of the prosthesis (100, 600A, 600B, 600C, 800) within the target blood vessel to thereby transition at least the elongated mid-portion (126) into the radially-expanded configuration.

2. The prosthesis of claim 1, wherein the channel (118, 118a, 118b, 118c, 418, 618A, 618B, 618C, 818) is a plurality of channels (118, 118a, 118b, 118c, 418, 618A, 618B, 618C, 818) formed in the wall (112, 112C, 812) of the elongated mid-portion (126), and wherein the second volume increases hydrostatic pressure within the plurality of channels (118, 118a, 118b, 118c, 418, 618A, 618B, 618C, 818) to produce a structural scaffold (116, 116C, 416, 816) about the elongated mid-portion (126); and optionally
wherein the tubular body (110, 110C, 610A, 610B, 610C, 810) defines a lumen (120, 820) through which blood may flow, and wherein the structural scaffold (116, 116C, 416, 816) provides buckling resistance at the elongated mid-portion (126).

3. The prosthesis of claim 1, wherein the tubular body (110, 110C, 610A, 610B, 610C, 810) comprises a flexible sheet (302, 302C) having opposing inner (304, 304C) and outer (306, 306C) layers that form the wall (112, 112C, 812) of the tubular body (110, 110C, 610A, 610B, 610C, 810) and between which the channel (118, 118a, 118b, 118c, 418, 618A, 618B, 618C, 818) is defined.

4. The prosthesis of claim 3, wherein the inner (304, 304C) and outer (306, 306C) layers are selected from one or more of polytetrafluoroethylene (PTFE), expanded PTFE (ePTFE), ultra-high-molecular-weight polyethylene (UHMWPE), polyurethane and polyester; and optionally
wherein the inner (304, 304C) and outer (306, 306C) layers comprise different materials.

5. The prosthesis of claim 1, wherein the fluid-absorbable composition (310, 410, 510) is a hydrogel or hydrophilic foam; or wherein the channel (118, 118a, 118b, 118c, 418, 618A, 618B, 618C, 818) is formed in one of a circumferential ring, a diamond pattern, a chevron pattern, a crisscross pattern, a spiral pattern and a sinusoidal pattern about the elongated mid-portion (126).

6. The prosthesis of claim 1, further comprising:
one or more wires (502) disposed within the channel (118, 118a, 118b, 118c, 418, 618A, 618B, 618C, 818), wherein the fluid-absorbable composition (310, 410, 510) at least partially surrounds the wire (502).

7. The prosthesis of claim 1, wherein the prosthesis (100, 600A, 600B, 600C, 800) is configured to substantially cover an enlarged area or cavity in the target blood vessel when the prosthesis (100, 600A, 600B, 600C, 800) is in the radially-expanded configuration; and optionally
wherein the enlarged area or cavity is an abdominal aortic aneurism or a thoracic aortic aneurysm, and wherein the prosthesis (100, 600A, 600B, 600C, 800) is implanted in the aorta in a manner to occlude the aneurism.

8. An expandable prosthetic device (100, 600A, 600B, 600C, 800) for implantation at a target blood vessel region to treat a target tissue defect in a patient, the device comprising:
a tubular body (110, 110C, 610A, 610B, 610C, 810) formed of graft material (114), the tubular body (110, 110C, 610A, 610B, 610C, 810) having a wall (112, 112C, 812) between first (121, 821) and second ends (125, 825) and a lumen (120, 820) defined by the wall (112, 112C, 812);
a self-expanding anchor stent (130) coupled to the first end (121, 821) for anchoring within the target blood vessel region when the device is implanted; and
a plurality of expandable flanges (402) arranged on an outer surface (404) of the wall (112, 112C, 812) of the tubular body (110, 110C, 610A, 610B, 610C, 810) in a geometric pattern, wherein each expandable flange (402) includes
an encapsulation material (406) coupled to the outer surface (404) of the wall (112, 112C, 812) for forming a channel (118, 118a, 118b, 118c, 418, 618A, 618B, 618C, 818) therebetween, and
a fluid-absorbable composition (310, 410, 510) contained within the channel (118, 118a, 118b, 118c, 418, 618A, 618B, 618C, 818), wherein the fluid-absorbable composition (310, 410, 510) at least partially swells upon exposure to bodily fluids *in situ,*
wherein at least partial swelling of the fluid-absorbable composition (310, 410, 510) within the channel (118, 118a, 118b, 118c, 418, 618A, 618B, 618C, 818) aids in radial expansion of the tubular body (110, 110C, 610A, 610B, 610C, 810).

9. The device of claim 8, wherein the encapsulation material (406) is coupled to the outer surface (404) of the wall (112, 112C, 812) to define tubes (418) configured to limit the swelling of the fluid-absorbable composition (310, 410, 510) therein; and optionally
wherein the flanges (402) provide turgid support structures (340, 440) when the fluid-absorbable composition (310, 410, 510) swells within the tubes (418), and wherein the turgid support structures (340, 440) are configured to at least provide an outward radial strength to the wall (112, 112C, 812) of the tubular body (110, 110C, 610A, 610B, 610C, 810).

10. The device of claim 8, wherein the lumen (120, 820) provides a passage through which blood may flow when the at least partial swelling of the fluid-absorbable composition (310, 410, 510) provides radial expansion of the tubular body (110, 110C, 610A, 610B, 610C, 810).

11. The device of claim 8, wherein the geometric pattern on the outer surface (404) of the wall (112, 112C, 812) of the tubular body (110, 110C, 610A, 610B, 610C, 810) includes at least one of longitudinally-spaced apart circumferential rings, a diamond pattern, a chevron pattern, a crisscross pattern, a spiral pattern and a sinusoidal pattern; and optionally wherein a central axis through the circumferential rings is substantially parallel to a longitudinal axis (201) of the tubular body (110, 110C, 610A, 610B, 610C, 810).

12. The device of claim 8, wherein one or more flanges (402) provide a radial force against the inner wall of the target blood vessel region.

13. The device of claim 8, wherein:
the graft material (114) is one of polyester and polyethylene terephthalate;
the encapsulation material (406) is one of ePTFE, polyurethane and polyester; and
the fluid-absorbable composition (310, 410, 510) is a hydrogel; or
wherein the target tissue defect is an abdominal aortic aneurism or a thoracic aortic aneurism, and wherein the device (100, 600A, 600B, 600C, 800) is implanted in the aorta in a manner to occlude the aneurism.

14. The prosthesis of claim 1, further comprising:
a branch stent-graft for directing fluid flow to a branch vessel from the target blood vessel.

15. The prosthesis of claim 1, further comprising:
a bifurcated portion having first and second tubular legs (128b, 128c) coupled to the second end (125, 825) of the tubular body (110, 110C, 610A, 610B, 610C, 810), wherein:
the first and second tubular legs (128b, 128c) define lumens that are in fluid communication with a lumen (120, 820) defined by the tubular body (110, 110C, 610A, 610B, 610C, 810), and
the tubular body (110, 110C, 610A, 610B, 610C, 810) is configured for placement within the abdominal aorta and the first and second tubular legs (128b, 128c) are configured for left and right iliac artery placement; or
wherein the anchoring structure (130) includes a plurality of crowns (132a, 132b) and a plurality of struts (134) with each crown (132a, 132b) being formed between a pair of opposing struts (134), wherein a first proximal-most set of crowns (132a, 132b) extend beyond a first edge of the tubular body (110, 110C, 610A, 610B, 610C, 810) and a second opposing set of crowns (132a, 132b) is coupled to the first end (121, 821) of the tubular body (110, 110C, 610A, 610B, 610C, 810).

## Patentansprüche

1. Prothese (100, 600A, 600B, 600C, 800), die eine komprimierte Konfiguration zum Abgeben innerhalb eines Gefäßsystems und eine radial erweiterte Konfiguration für einen Einsatz innerhalb eines Zielblutgefäßes in einem Patienten aufweist, die Prothese umfassend:
einen röhrenförmigen Körper (110, 110C, 610A, 610B, 610C, 810), der aufweist
ein erstes Ende (121, 821) und ein zweites Ende (125, 825), wobei das erste Ende (121, 821) eine Verankerungsstruktur aufweist, um eine Innenwand des Zielblutgefäßes in der radial erweiterten Konfiguration in Eingriff zu nehmen; und
einen länglichen Mittelabschnitt (126) zwischen dem ersten (121, 821) und dem zweiten Ende (125, 825) und der einen Kanal (118, 118a, 118b, 118c, 418, 618A, 618B, 618C, 818) einschließt, der in einer Wand (112, 112C, 812) davon ausgebildet ist, wobei der Kanal (118, 118a, 118b, 118c, 418, 618A, 618B, 618C, 818) mindestens teilweise um den röhrenförmigen Körper (110, 110C, 610A, 610B, 610C, 810) herum ausgerichtet ist; und
eine fluidabsorbierbare Zusammensetzung (310, 410, 510), die innerhalb des Kanals (118, 118a, 118b, 118c, 418, 618A, 618B, 618C, 818) abgeschieden ist, wobei die fluidabsorbierbare Zusammensetzung (310, 410, 510) ein erstes Volumen aufweist, wenn sich die Prothese (100, 600A, 600B, 600C, 800) in der komprimierten Konfiguration befindet und konfiguriert ist, um auf ein zweites Volumen innerhalb des Kanals (118, 118a, 118b, 118c, 418, 618A, 618B, 618C, 818) bei einem Einsetzen der Prothese (100, 600A, 600B, 600C, 800) innerhalb des Zielblutgefäßes anzuschwellen, um dadurch mindestens den länglichen Mittelabschnitt (126) in die radial erweiterte Konfiguration zu überführen.

2. Prothese nach Anspruch 1, wobei der Kanal (118, 118a, 118b, 118c, 418, 618A, 618B, 618C, 818) eine Vielzahl von Kanälen (118, 118a, 118b, 118c, 418, 618A, 618B, 618C, 818) ist, die in der Wand (112, 112C, 812) des länglichen Mittelabschnitts (126) ausgebildet sind, und wobei das zweite Volumen einen hydrostatischen Druck innerhalb der Vielzahl von Kanälen (118, 118a, 118b, 118c, 418, 618A, 618B, 618C, 818) erhöht, um ein Strukturgerüst (116, 116C, 416, 816) um den länglichen Mittelabschnitt (126) herum zu erzeugen; und optional
wobei der röhrenförmige Körper (110, 110C, 610A, 610B, 610C, 810) ein Lumen (120, 820) definiert, durch das Blut fließen kann, und wobei das Strukturgerüst (116, 116C, 416, 816) eine Knickbeständigkeit an dem länglichen Mittelabschnitt (126) bereitstellt.

3. Prothese nach Anspruch 1, wobei der röhrenförmige Körper (110, 110C, 610A, 610B, 610C, 810) eine flexible Bahn (302, 302C) aufweist, die gegenüberliegende innere (304, 304C) und äußere (306, 306C) Schichten aufweist, die die Wand (112, 112C, 812) des röhrenförmigen Körpers (110, 110C, 610A, 610B, 610C, 810) ausbilden und zwischen denen der Kanal (118, 118a, 118b, 118c, 418, 618A, 618B, 618C, 818) definiert ist.

4. Prothese nach Anspruch 3, wobei die inneren (304, 304C) und die äußeren (306, 306C) Schichten aus einem oder mehreren von Polytetrafluorethylen (PTFE), erweitertem PTFE (ePTFE), Polyethylen mit ultrahohem Molekulargewicht (UHMWPE), Polyurethan und Polyester ausgewählt sind; und optional
wobei die inneren (304, 304C) und die äußeren (306, 306C) Schichten unterschiedliche Materialien umfassen.

5. Prothese nach Anspruch 1, wobei die fluidabsorbierbare Zusammensetzung (310, 410, 510) ein Hydrogel oder ein hydrophiler Schaum ist; oder wobei der Kanal (118, 118a, 118b, 118c, 418, 618A, 618B, 618C, 818) in einem von einem Umfangsring, einem Diamantmuster, einem Chevron-Muster, einem Kreuzmuster, einem Spiralmuster und einem sinusförmigen Muster um den länglichen Mittelabschnitt (126) herum ausgebildet ist.

6. Prothese nach Anspruch 1, ferner umfassend:
einen oder mehrere Drähte (502), die innerhalb des Kanals (118, 118a, 118b, 118c, 418, 618A, 618B, 618C, 818) angeordnet sind, wobei die fluidabsorbierbare Zusammensetzung (310, 410, 510) den Draht (502) mindestens teilweise umgibt.

7. Prothese nach Anspruch 1, wobei die Prothese (100, 600A, 600B, 600C, 800) konfiguriert ist, um im Wesentlichen einen vergrößerten Bereich oder Hohlraum in dem Zielblutgefäß abzudecken, wenn sich die Prothese (100, 600A, 600B, 600C, 800) in der radial erweiterten Konfiguration befindet; und optional
wobei der vergrößerte Bereich oder Hohlraum ein Bauchaortenaneurysma oder ein Thoraxaortenaneurysma ist, und wobei die Prothese (100, 600A, 600B, 600C, 800) in die Aorta auf eine Weise implantiert ist, um das Aneurysma zu verschließen.

8. Erweiterbare prothetische Vorrichtung (100, 600A, 600B, 600C, 800) für eine Implantation an einem Zielblutgefäßbereich, um einen Zielgewebedefekt in einem Patienten zu behandeln, die Vorrichtung umfassend:
einen röhrenförmigen Körper (110, 110C, 610A, 610B, 610C, 810), der aus Transplantatmaterial (114) ausgebildet ist, wobei der röhrenförmige Körper (110, 110C, 610A, 610B, 610C, 810) eine Wand (112, 112C, 812) zwischen ersten (121, 821) und zweiten Enden (125, 825) und ein Lumen (120, 820) aufweist, das durch die Wand (112, 112C, 812) definiert ist;
einen selbsterweiterbaren Ankerstent (130), der mit dem ersten Ende (121, 821) gekoppelt ist, zum Verankern innerhalb des Zielblutgefäßbereichs, wenn die Vorrichtung implantiert ist; und
eine Vielzahl von erweiterbaren Flanschen (402), die auf einer Außenoberfläche (404) der Wand (112, 112C, 812) des röhrenförmigen Körpers (110, 110C, 610A, 610B, 610C, 810) in einem geometrischen Muster angeordnet sind, wobei jeder erweiterbare Flansch (402) einschließt
ein Verkapselungsmaterial (406), das mit der Außenoberfläche (404) der Wand (112, 112C, 812) gekoppelt ist, zum Ausbilden eines Kanals (118, 118a, 118b, 118c, 418, 618A, 618B, 618C, 818) dazwischen, und
eine fluidabsorbierbare Zusammensetzung (310, 410, 510), die innerhalb des Kanals (118, 118a, 118b, 118c, 418, 618A, 618B, 618C, 818) enthalten ist, wobei die fluidabsorbierbare Zusammensetzung (310, 410, 510) bei einer Exposition gegenüber Körperflüssigkeiten *in situ* mindestens teilweise anschwillt,
wobei mindestens teilweises Anschwellen der fluidabsorbierbaren Zusammensetzung (310, 410, 510) innerhalb des Kanals (118, 118a, 118b, 118c, 418, 618A, 618B, 618C, 818) bei der radialen Erweiterung des röhrenförmigen Körpers (110, 110C, 610A, 610B, 610C, 810) unterstützt.

9. Vorrichtung nach Anspruch 8, wobei das Verkapselungsmaterial (406) mit der Außenoberfläche (404) der Wand (112, 112C, 812) gekoppelt ist, um Röhren (418) zu definieren, die konfiguriert sind, um das Schwellen der fluidabsorbierbaren Zusammensetzung (310, 410, 510) darin zu begrenzen; und optional
wobei die Flansche (402) aufgedunsene Stützstrukturen (340, 440) bereitstellen, wenn die fluidabsorbierbare Zusammensetzung (310, 410, 510) innerhalb der Röhren (418) anschwillt, und wobei die aufgedunsenen Stützstrukturen (340, 440) konfiguriert sind, um mindestens eine nach außen gerichtete radiale Festigkeit an die Wand (112, 112C, 812) des röhrenförmigen Körpers (110, 110C, 610A, 610B, 610C, 810) bereitzustellen.

10. Vorrichtung nach Anspruch 8, wobei das Lumen (120, 820) einen Durchgang bereitstellt, durch den Blut fließen kann, wenn das mindestens teilweise Anschwellen der fluidabsorbierbaren Zusammensetzung (310, 410, 510) eine radiale Erweiterung des röhrenförmigen Körpers (110, 110C, 610A, 610B, 610C, 810) bereitstellt.

11. Vorrichtung nach Anspruch 8, wobei das geometrische Muster auf der Außenoberfläche (404) der Wand (112, 112C, 812) des röhrenförmigen Körpers (110, 110C, 610A, 610B, 610C, 810) mindestens eines von in Längsrichtung beabstandeten Umfangsringen, einem Diamantmuster, einem Chevron-Muster, einem Kreuzmuster, einem Spiralmuster und einem sinusförmigen Muster einschließt; und optional wobei eine Mittelachse durch die Umfangsringe im Wesentlichen parallel zu einer Längsachse (201) des röhrenförmigen Körpers (110, 110C, 610A, 610B, 610C, 810) ist.

12. Vorrichtung nach Anspruch 8, wobei ein oder mehrere Flansche (402) eine radiale Kraft gegen die Innenwand des Zielblutgefäßbereichs bereitstellen.

13. Vorrichtung nach Anspruch 8, wobei:
das Transplantatmaterial (114) eines von Polyester und Polyethylenterephthalat ist;
das Verkapselungsmaterial (406) eines aus ePTFE, Polyurethan und Polyester ist; und die fluidabsorbierbare Zusammensetzung (310, 410, 510) ein Hydrogel ist; oder
wobei der Zielgewebedefekt ein Bauchaortenaneurysma oder ein Thoraxaortenaneurysma ist, und wobei die Vorrichtung (100, 600A, 600B, 600C, 800) in die Aorta auf eine Weise implantiert ist, um das Aneurysma zu verschließen.

14. Prothese nach Anspruch 1, ferner umfassend:
ein Abzweigstenttransplantat zum Leiten eines Fluidstroms zu einem Zweiggefäß von dem Zielblutgefäß.

15. Prothese nach Anspruch 1, ferner umfassend:
einen gegabelten Abschnitt, der einen ersten und einen zweiten röhrenförmigen Schenkel (128b, 128c) aufweist, die mit dem zweiten Ende (125, 825) des röhrenförmigen Körpers (110, 110C, 610A, 610B, 610C, 810) gekoppelt sind, wobei:
der erste und der zweite röhrenförmige Schenkel (128b, 128c) Lumen definieren, die in Fluidverbindung mit einem Lumen (120, 820) stehen, das durch den röhrenförmigen Körper (110, 110C, 610A, 610B, 610C, 810) definiert ist, und
der röhrenförmige Körper (110, 110C, 610A, 610B, 610C, 810) für die Platzierung innerhalb der Bauchaorta konfiguriert ist und der erste und der zweite röhrenförmige Schenkel (128b, 128c) für linke und rechte iliakale arterielle Platzierung konfiguriert sind; oder
wobei die Verankerungsstruktur (130) eine Vielzahl von Kronen (132a, 132b) und eine Vielzahl von Streben (134) einschließt, wobei jede Krone (132a, 132b) zwischen einem Paar gegenüberliegender Streben (134) ausgebildet ist, wobei sich ein erster proximalster Satz von Kronen (132a, 132b) über eine erste Kante des röhrenförmigen Körpers (110, 110C, 610A, 610B, 610C, 810) hinaus erstreckt und ein zweiter gegenüberliegender Satz von Kronen (132a, 132b) mit dem ersten Ende (121, 821) des röhrenförmigen Körpers (110, 110C, 610A, 610B, 610C, 810) gekoppelt ist.

## Revendications

1. Prothèse (100, 600A, 600B, 600C, 800) ayant une configuration comprimée permettant l'administration à l'intérieur d'un système vasculaire et une configuration radialement étendue pour le déploiement à l'intérieur d'un vaisseau sanguin cible chez un patient, la prothèse comprenant :
un corps tubulaire (110, 110C, 610A, 610B, 610C, 810) ayant
une première extrémité (121, 821) et une seconde extrémité (125, 825), la première extrémité (121, 821) ayant une structure d'ancrage pour venir en prise avec une paroi interne du vaisseau sanguin cible dans la configuration radialement étendue ; et
une partie médiane allongée (126) entre les première (121, 821) et seconde extrémités (125, 825) et comportant un canal (118, 118a, 118b, 118c, 418, 618A, 618B, 618C, 818) formé dans une paroi (112, 112C, 812) de celui-ci, dans laquelle le canal (118, 118a, 118b, 118c, 418, 618A, 618B, 618C, 818) est au moins partiellement orienté de manière circonférentielle autour du corps tubulaire (110, 110C, 610A, 610B, 610C, 810) ; et
une composition résorbable par fluide (310, 410, 510) déposée à l'intérieur du canal (118, 118a, 118b, 118c, 418, 618A, 618B, 618C, 818), la composition résorbable par fluide (310, 410, 510) ayant un premier volume lorsque la prothèse (100, 600A, 600B, 600C, 800) est dans la configuration comprimée et conçue pour gonfler à un second volume à l'intérieur du canal (118, 118a, 118b, 118c, 418, 618A, 618B, 618C, 818) lors du déploiement de la prothèse (100, 600A, 600B, 600C, 800) dans le vaisseau sanguin cible pour ainsi faire passer au moins la partie médiane allongée (126) dans la configuration radialement étendue.

2. Prothèse selon la revendication 1, dans laquelle le canal (118, 118a, 118b, 118c, 418, 618A, 618B, 618C, 818) est une pluralité de canaux (118, 118a, 118b, 118c, 418, 618A, 618B, 618C, 818) formés dans la paroi (112, 112C, 812) de la partie médiane allongée (126), et dans laquelle le second volume augmente la pression hydrostatique à l'intérieur de la pluralité de canaux (118, 118a, 118b, 118c, 418, 618A, 618B, 618C, 818) pour produire un échafaudage structurel (116, 116C, 416, 816) autour de la partie médiane allongée (126) ; et éventuellement
dans laquelle le corps tubulaire (110, 110C, 610A, 610B, 610C, 810) définit une lumière (120, 820) à travers laquelle du sang peut s'écouler, et dans laquelle l'échafaudage structurel (116, 116C, 416, 816) fournit une résistance au flambage au niveau de la partie médiane allongée (126).

3. Prothèse selon la revendication 1, dans laquelle le corps tubulaire (110, 110C, 610A, 610B, 610C, 810) comprend une feuille flexible (302, 302C) ayant des couches internes (304, 304C) et externes (306, 306C) opposées qui forment la paroi (112, 112C, 812) du corps tubulaire (110, 110C, 610A, 610B, 610C, 810) et entre lesquelles le canal (118, 118a, 118b, 118c, 418, 618A, 618B, 618C, 818) est défini.

4. Prothèse selon la revendication 3, dans laquelle les couches internes (304, 304C) et externes (306, 306C) sont choisies parmi un ou plusieurs parmi le polytétrafluoroéthylène (PTFE), le PTFE expansé (ePTFE), le polyéthylène à poids moléculaire ultra-élevé (UHMWPE), le polyuréthane et le polyester ; et éventuellement
dans laquelle les couches internes (304, 304C) et externes (306, 306C) comprennent des matériaux différents.

5. Prothèse selon la revendication 1, dans laquelle la composition résorbable par fluide (310, 410, 510) est une mousse hydrogel ou hydrophile ; ou dans laquelle le canal (118, 118a, 118b, 118c, 418, 618A, 618B, 618C, 818) est formé dans l'un parmi un anneau circonférentiel, un motif diamant, un motif en chevron, un motif entrecroisé, un motif en spirale et un motif sinusoïdal autour de la partie médiane allongée (126).

6. Prothèse selon la revendication 1, comprenant en outre :
un ou plusieurs fils (502) disposés à l'intérieur du canal (118, 118a, 118b, 118c, 418, 618A, 618B, 618C, 818), dans laquelle la composition résorbable par fluide (310, 410, 510) entoure au moins partiellement le fil (502).

7. Prothèse selon la revendication 1, dans laquelle la prothèse (100, 600A, 600B, 600C, 800) est conçue pour couvrir sensiblement une zone ou une cavité élargie dans le vaisseau sanguin cible lorsque la prothèse (100, 600A, 600B, 600C, 800) est dans la configuration radialement étendue ; et éventuellement
dans laquelle la zone ou la cavité élargie est un anévrisme aortique abdominal ou un anévrisme aortique thoracique, et dans laquelle la prothèse (100, 600A, 600B, 600C, 800) est implantée dans l'aorte de manière à occlure l'anévrisme.

8. Dispositif prothétique expansible (100, 600A, 600B, 600C, 800) permettant une implantation au niveau d'une région cible de vaisseau sanguin pour traiter un défaut tissulaire cible chez un patient, le dispositif comprenant :
un corps tubulaire (110, 110C, 610A, 610B, 610C, 810) formé de matériau de greffe (114), le corps tubulaire (110, 110C, 610A, 610B, 610C, 810) ayant une paroi (112, 112C, 812) entre des première (121, 821) et seconde extrémités (125, 825) et une lumière (120, 820) définie par la paroi (112, 112C, 812) ;
une endoprothèse d'ancrage auto-expansible (130) accouplée à la première extrémité (121, 821) pour s'ancrer à l'intérieur de la région cible de vaisseau sanguin lorsque le dispositif est implanté ; et
une pluralité de brides expansibles (402) agencées sur une surface externe (404) de la paroi (112, 112C, 812) du corps tubulaire (110, 110C, 610A, 610B, 610C, 810) dans un motif géométrique, dans lequel chaque bride expansible (402) comporte
un matériau d'encapsulation (406) accouplé à la surface externe (404) de la paroi (112, 112C, 812) pour former un canal (118, 118a, 118b, 118c, 418, 618A, 618B, 618C, 818) entre eux, et
une composition résorbable par fluide (310, 410, 510) contenue à l'intérieur du canal (118, 118a, 118b, 118c, 418, 618A, 618B, 618C, 818), dans lequel la composition résorbable par fluide (310, 410, 510) gonfle au moins partiellement lors de l'exposition à des fluides corporels *in situ,*
dans lequel au moins un gonflement partiel de la composition résorbable par fluide (310, 410, 510) à l'intérieur du canal (118, 118a, 118b, 118c, 418, 618A, 618B, 618C, 818) aide à l'expansion radiale du corps tubulaire (110, 110C, 610A, 610B, 610C, 810).

9. Dispositif selon la revendication 8, dans lequel le matériau d'encapsulation (406) est accouplé à la surface externe (404) de la paroi (112, 112C, 812) pour définir des tubes (418) conçus pour limiter le gonflement de la composition résorbable par fluide (310, 410, 510) à l'intérieur de celle-ci ; et éventuellement
dans lequel les brides (402) fournissent des structures de support turgescentes (340, 440) lorsque la composition résorbable par fluide (310, 410, 510) gonfle à l'intérieur des tubes (418), et dans lequel les structures de support turgescentes (340, 440) sont conçues pour au moins fournir une force radiale vers l'extérieur à la paroi (112, 112C, 812) du corps tubulaire (110, 110C, 610A, 610B, 610C, 810).

10. Dispositif selon la revendication 8, dans lequel la lumière (120, 820) fournit un passage à travers lequel le sang peut s'écouler lorsque le gonflement au moins partiel de la composition résorbable par fluide (310, 410, 510) fournit une expansion radiale du corps tubulaire (110, 110C, 610A, 610B, 610C, 810).

11. Dispositif selon la revendication 8, dans lequel le motif géométrique sur la surface externe (404) de la paroi (112, 112C, 812) du corps tubulaire (110, 110C, 610A, 610B, 610C, 810) comporte au moins l'un parmi des anneaux circonférentiels espacés longitudinalement, un motif diamant, un motif en chevron, un motif entrecroisé, un motif en spirale et un motif sinusoïdal ; et éventuellement dans lequel un axe central à travers les anneaux circonférentiels est sensiblement parallèle à un axe longitudinal (201) du corps tubulaire (110, 110C, 610A, 610B, 610C, 810).

12. Dispositif selon la revendication 8, dans lequel une ou plusieurs brides (402) fournissent une force radiale contre la paroi interne de la région du vaisseau sanguin cible.

13. Dispositif selon la revendication 8, dans lequel :
le matériau de greffe (114) est l'un parmi le polyester et le polyéthylène téréphtalate ;
le matériau d'encapsulation (406) est l'un parmi l'ePTFE, le polyuréthane et le polyester ; et la composition résorbable par fluide (310, 410, 510) est un hydrogel ; ou
dans lequel le défaut de tissu cible est un anévrisme aortique abdominal ou un anévrisme aortique thoracique, et dans lequel le dispositif (100, 600A, 600B, 600C, 800) est implanté dans l'aorte de manière à occlure l'anévrisme.

14. Prothèse selon la revendication 1, comprenant en outre :
une endoprothèse de branche pour diriger un écoulement de fluide vers un vaisseau de branche à partir du vaisseau sanguin cible.

15. Prothèse selon la revendication 1, comprenant en outre :
une partie bifurquée ayant des première et seconde pattes tubulaires (128b, 128c) accouplées à la seconde extrémité (125, 825) du corps tubulaire (110, 110C, 610A, 610B, 610C, 810), dans laquelle :
les première et seconde pattes tubulaires (128b, 128c) définissent des lumières qui sont en communication fluidique avec une lumière (120, 820) définie par le corps tubulaire (110, 110C, 610A, 610B, 610C, 810), et
le corps tubulaire (110, 110C, 610A, 610B, 610C, 810) est conçu pour être placé à l'intérieur de l'aorte abdominale et les première et seconde pattes tubulaires (128b, 128c) sont conçues pour un placement d'artère iliaque gauche et droite ; ou
dans laquelle la structure d'ancrage (130) comporte une pluralité de couronnes (132a, 132b) et une pluralité d'entretoises (134) avec chaque couronne (132a, 132b) formée entre une paire d'entretoises opposées (134), dans laquelle un premier ensemble de couronnes le plus proximal (132a, 132b) s'étend au-delà d'un premier bord du corps tubulaire (110, 110C, 610A, 610B, 610C, 810) et un second ensemble opposé de couronnes (132a, 132b) étant accouplé à la première extrémité (121, 821) du corps tubulaire (110, 110C, 610A, 610B, 610C, 810).
